(19) Europäisches Patentamt / European Patent Office / Office européen des brevets

(11) **EP 2 848 194 B1**

(12) **EUROPEAN PATENT SPECIFICATION**

(45) Date of publication and mention
of the grant of the patent:
**27.04.2016  Bulletin 2016/17**

(51) Int Cl.:
*A61B 5/024* *(2006.01)*    *A61B 5/00* *(2006.01)*

(21) Application number: **14182467.2**

(22) Date of filing: **27.08.2014**

(54) **Method, system and apparatus for generating pulse estimate**

Verfahren, System und Vorrichtung zur Erzeugung von Impulsschätzung

Procédé, système et appareil pour générer une estimation d'impulsion

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.09.2013  FI 20135901**

(43) Date of publication of application:
**18.03.2015  Bulletin 2015/12**

(73) Proprietor: **TreLab**
**33100 Tampere (FI)**

(72) Inventors:
- **Forsell, Vili**
  **33720 Tampere (FI)**
- **Kärkkäinen, Tero**
  **39130 Sasi (FI)**

(74) Representative: **Espatent Oy**
**Kaivokatu 10 D**
**00100 Helsinki (FI)**

(56) References cited:
WO-A1-2012/068613    US-A1- 2009 326 395
US-A1- 2013 079 606

**Description**

TECHNICAL FIELD

The present application generally relates to a method, a system and an apparatus for generating a pulse estimate.

BACKGROUND ART

World of health care faces many challenges. Despite an intense focus on the safety and quality of the health care provided e.g. in hospitals, progress is frustratingly slow. One weakness in our health care systems is the lack of simple and cost-efficient assessment of a person's possible health risks that can be communicated as early as possible to healthcare professionals in order to expedite treatment in critical conditions. In health care environment an early indication of a change in the patient's health status is critical. The earlier the indications can be forwarded to the health care professionals, the better. Automated health evaluation systems are especially important, since sometimes not even the patient herself notices significant changes in her condition. Current nursing staffing and physician-coverage practices have accentuated the need for a tool that can highlight the changes in a patient's health that are not easily apparent to a caregiver with an unfamiliar patient.

US 2009/326395 discloses a method for detecting pulses in a PPG signal. Local minima and maxima points may be identified in the PPG signal. Each minimum may be paired with an adjacent maximum forming an upstroke segment. Noise may be removed by comparing adjacent segments and ignoring segments that are too long or too large. Notches in the pulse may be identified and ignored by analyzing adjacent segments. Adjacent upstroke segments may be combined as a single upstroke if the lengths of adjacent upstroke segments are about the same, have similar slopes, and the end point of one segment is close to the start point of an adjacent segment. Segments having small temporal or amplitude difference relative to adjacent segments may be disregarded. The remaining segments may represent the pulse upticks. A sliding time window may be used instead to detect pulses in the PPG signal.

Hospitals provide professional and expensive systems to health care operations. They are insufficient due to overcrowding and an increased number of older people that require monitoring in a home environment with no access to hospital equipment. Because of resource limitations, the number of patients that can be monitored and treated in intensive care units and high dependency units is restricted. The selection of patients who might benefit from critical care is therefore crucial. Identifying medical in-patients at risk of deterioration in an early stage by means of simple protocols based on physiological parameters may reduce the number of pre-ICU resuscitations.

There is clearly a need for a new solution to provide an easy and low-cost system for detecting biometrical information in real-time and to recognize the status of the person, such as pulse or temperature, for example. Furthermore, improved methods for connectivity and sharing the information are needed to rapidly transmit the health risks to the healthcare professionals and, thus, hasten the treatment of rapidly worsening conditions.

SUMMARY

The present invention is defined in the appended claims.

According to the invention there is provided a portable apparatus comprising:

a communication interface for communicating with a wireless network;
a photo sensor for receiving non-invasive biometric information of a user;
at least one processor; and
at least one memory including computer program code; the at least one memory and the computer program code configured to, with the at least one processor, cause the portable apparatus to:

transmit a light beam using the light source;
receive noisy biometric information using a photo sensor; wherein the received noisy biometric information comprises a reflection of the transmitted light beam;
filter the noisy biometric information to provide filtered input data for reducing noise;
derive the input data to provide local maxima values of the input data;
define at least one local maxima value pair;
determine a weight value for the at least one local maxima value pair;
select a local maxima value pair of the highest weight value; and
generate an estimate of a pulse based on the selected local maxima value pair.

In an embodiment, filtering the noisy biometric information comprises at least one of the following:

resample the biometric information;
noise reduce filter the biometric information;
all-pass filter (APF) the biometric information;
bin the biometric information;
low-pass filter (LPF) the biometric information;
high-pass filter (HPF) the biometric information; and
band-pass filter (BPF) the biometric information.

In an embodiment, median and moving average filters may not attenuate certain frequencies, but help reduce noise. All-pass filters are another case where the target may not be to focus on silencing a certain frequency component.
In an embodiment, the at least one memory and the computer program code configured to, with the at least one processor, cause the portable apparatus to:

generate an estimate of a pulse based on the difference of the local maxima values of the selected local maxima value pair.

In an embodiment, the portable apparatus further comprising at least one light source, wherein the at least one memory and the computer program code configured to, with the at least one processor, cause the portable apparatus to:

transmit a light beam using the light source; and
receive a noisy biometric information using a photo sensor, wherein the received noisy biometric information comprising a reflection of the transmitted light beam.

In an embodiment, the photo sensor comprises a photodiode.
In an embodiment, the noisy biometric information is received from a human body.
In an embodiment, the light source comprises a light emitting diode.
In an embodiment, the at least one memory and the computer program code configured to, with the at least one processor, cause the portable apparatus to:

transmit the estimate of the pulse to a server apparatus over a wireless network.

In an embodiment, the at least one memory and the computer program code configured to, with the at least one processor, cause the portable apparatus to:

provide metadata associated to the estimate of a pulse.

In an embodiment, the metadata comprising at least one of the following:

temperature information;
pressure information;
movement information;
location information; and
humidity information.

In an embodiment, the received noisy biometric information comprises information on arterial blood volume changes caused by pumping actions of a heart.
In an embodiment, the at least one memory and the computer program code configured to, with the at least one processor, cause the portable apparatus to:

filter the noisy biometric information to provide filtered input data for reducing noise.

In an embodiment, the at least one memory and the computer program code configured to, with the at least one processor, cause the portable apparatus to:

derive the filtered input data to provide local maxima values; and
define a size of the local maxima value as a difference between the local maxima value and a preceding local minimum value of the filtered input data.

In an embodiment, the at least one memory and the computer program code configured to, with the at least one processor, cause the portable apparatus to:

bin the noisy biometric information to provide binned input data with median representatives.

In an embodiment, the at least one memory and the computer program code configured to, with the at least one processor, cause the portable apparatus to:

derive the median representatives of binned input data to provide a local maxima value; and
define a size of the local maxima value as a difference between the local maxima value and a preceding local minimum value of the binned input data.

According to an example there is provided a system comprising:

a portable apparatus of the invention; and
a server apparatus comprising:

a communication interface for communicating with a wireless network;
at least one processor; and
at least one memory including computer program code; the at least one memory and the computer program code configured to, with the at least one processor, cause the server apparatus to:

receive an estimate of a pulse;
maintain user profile information for users of a server system service, comprising; and
trigger an action based on the received estimate of the pulse and the user profile information.

In an embodiment, the at least one memory and the computer program code configured to, with the at least one processor, cause the server apparatus to:

receive metadata associated to the estimate of a pulse.

In an embodiment, the metadata comprising at least one of the following:

temperature information;
pressure information;
movement information;
location information; and
humidity information.

In an embodiment, the at least one memory and the computer program code configured to, with the at least one processor, cause the server apparatus to:

transmit the estimate of the pulse to a remote apparatus over the wireless network in response to the triggered action.

According to the invention there is provided a method comprising:

receiving noisy biometric information using a photo sensor;
wherein the received noisy biometric information comprises a reflection of the transmitted light beam;
filtering the noisy biometric information to provide filtered input data for reducing noise;
deriving the input data to provide local maxima values of the input data;
defining at least one local maxima value pair;
determining a weight value for the at least one local maxima value pair;
selecting a local maxima value pair of the highest weight value; and
generating an estimate of a pulse based on the selected local maxima value pair.

According to the invention there is provided a computer program embodied on a computer readable medium comprising computer executable program code, which when executed by at least one processor of a portable apparatus, causes the portable apparatus to:

transmit a light beam using a light source;
receive noisy biometric information using a photo sensor; wherein the received noisy biometric information comprises a reflection of the transmitted light beam;
filter the noisy biometric information to provide filtered input data for noise reduction;
derive the input data to provide local maxima values of the input data;
define at least one local maxima value pair;
determine a weight value for the at least one local maxima value pair;
select a local maxima value pair of the highest weight value; and
generate an estimate of a pulse based on the selected local maxima value pair.

Different non-binding example aspects and embodiments of the present invention have been illustrated in the foregoing. Some embodiments may be presented only with reference to certain example aspects of the invention.

BRIEF DESCRIPTION OF THE DRAWINGS

The invention will be described, by way of example only, with reference to the accompanying drawings, in which:

Fig. 1   shows a schematic picture of a system according to an example embodiment of the invention;
Fig. 2   presents an example block diagram of a portable apparatus in which various embodiments of the invention may be applied;
Fig. 3   presents an example block diagram of a server apparatus in which various embodiments of the invention may be applied;
Fig. 4   presents an example block diagram of a portable apparatus for generating an estimate of a pulse in which various embodiments of the invention may be applied;
Fig. 5a   presents a schematic picture of binned input data peaks with median representatives in which various embodiments of the invention may be applied;
Fig. 5b   presents a schematic picture of model input data peaks in which various embodiments of the invention may be applied;
Fig.6a   presents a schematic picture of a graph representation with a lightest path using input data peaks and model data peaks in which various embodiments of the invention may be applied;
Fig.6b   presents a schematic picture of a graph representation including only interesting set switches in which various embodiments of the invention may be applied;
Fig.6c   presents a schematic picture of a graph representation with chosen minimal distance peak pairs in which various embodiments of the invention may be applied; and
Fig. 7   shows a flow diagram showing operations of the portable apparatus in accordance with an example embodiment of the invention.

DETAILED DESCRIPTION

The disclosed solution is intended for providing people improved care services. It is ideal for those who want to increase efficiency, create safer environments and offer better service level.

The solution consists of three main elements; cookie-sized portable apparatuses (tags), one or more wireless access points and a cloud service of server apparatus. It can be further complemented by a mobile application. The solution is simple and easy-to-use. The portable apparatus tracks presence, movement, and activity combined with the possibility to have additional nurse call and access control systems - all in one. With the solution, monitoring turns into preparedness as possible problems can be recognized early on.

Portable apparatuses are small, light and very discrete when carried. Patients and staff feel comfortable using them. The disclosed solution takes care of some of routines and allows staff to focus on critical tasks, resulting in better care and customer satisfaction. Using the service solution requires only minimal additional infrastructure set-up costs - broadband connectivity is all that is needed.

The portable apparatus makes critical, event-based sensor data gathering painless. The wireless measurement unit is small and light in physical size, resulting in non-disturbing measuring and unbiased measurement data. Despite the small size, the device is multi-functional - it is capable of capturing biometric information, presence/location, movement, force & direction information as well as measuring surrounding conditions - temperature, relative humidity, and atmospheric pressure. All functionality is available in one device.

The portable apparatus may be a wearable apparatus, such as a wrist watch kind of device. The portable apparatus may comprise a multiple of devices, such as a light source device and a photo sensor device.

The system has flexibility to be matched with typical customer back-end system requirements. Furthermore, the wireless measurement unit and its features can be optimized with the measuring conditions, e.g. to ensure optimal power consumption.

In the following description, like numbers denote like elements.

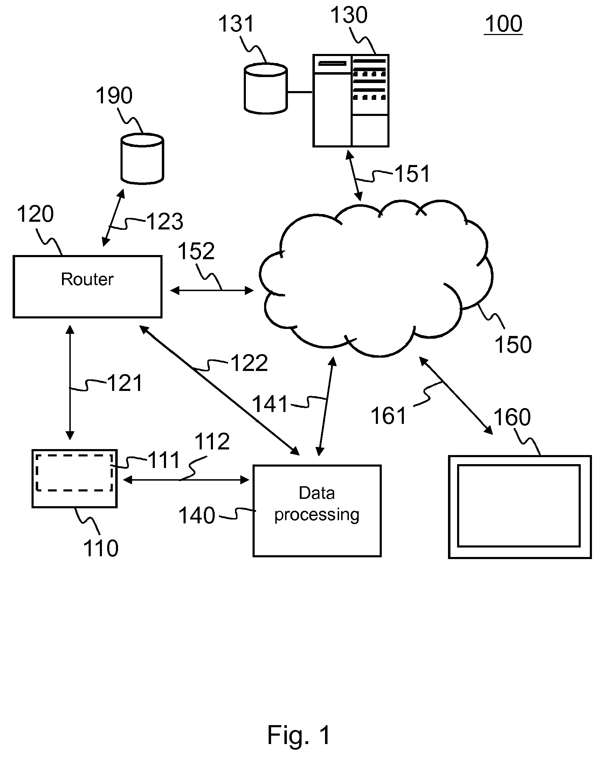

Fig. 1 shows a schematic picture of a system 100 according to an example embodiment of the invention.

The system 100 is configured to provide health care, environmental and security information maximizing the effects and minimizing the complexity and costs. A technical architecture of the system 100 is illustrated for components, their relations and communication between each other in addition to needed basic functionality in HW and SW. At the minimum, the system 100 comprises a portable apparatus 110 for providing the input data, a router 120 for providing the input data or the processed outcome of it for a network 150 and a server 130 providing a cloud service for the portable apparatus data. Optionally, further apparatuses may be added, such as peripheral devices for maintaining, providing or processing the portable apparatus data and communication devices for connecting the peripheral devices to the system 100.

A portable apparatus 110, such as a tag, may comprise a user interface 111 for a user or alternatively may not comprise user interface at all. The portable apparatus 110 is capable of locally executing software program code. The software program code may be a client application of a service whose processing application is running on a processing apparatus 140 of the system 100. The portable apparatus 110 is configured to be connectable to a router apparatus 120 over a local connection 121. The local connection 121 may comprise a wireless local area network (WLAN), Bluetooth, infrared (IR) connection, Radio Frequency Identification (RF-ID), near field communication (NFC) or a wireless non-cellular connection. The wireless non-cellular connection may comprise industrial, scientific and medical (ISM) radio bands that are radio bands (portions of the radio spectrum) reserved internationally for the use of radio frequency (RF) energy for industrial, scientific and medical purposes, for example. The portable apparatus 110 is configured to be connectable to a data processing apparatus 140 over a local connection 112. The local connection 112 may comprise a wireless local area network (WLAN), Bluetooth, infrared (IR) connection, Radio Frequency Identification (RF-ID), near field communication (NFC) or a wireless non-cellular connection.

The router apparatus 120 provides access between local apparatuses 110, 140, 190 and access between a wide area network 150, such as Internet, and the apparatuses 110, 130, 140, 160. The router apparatus provides local data connections 121, 122 for the portable apparatus 110 and a processing apparatus 140. The router apparatus 120 is connected to the wide area network 150, such as Internet, over a network connection 152. The network connection 152 may comprise a cable TV or an ADSL connection, for example.

In an embodiment, the portable apparatus 110 is configured to communicate to the network 150 over a cellular access. In such case the router 120 may correspond to the cellular sub-system, such as base stations and core network.

In an embodiment, a network attached storage (NAS) 190 may be connected to the router apparatus 120 as a data source. The network attached storage (NAS) may comprise data for the processing apparatus 140. The network attached storage (NAS) 190 is connected to the routing apparatus 120 via a local data connection 123. The local data connection 123 may comprise HDMI, USB, WLAN or Ethernet, for example.

The processing apparatus 140 may receive measurement data from different parts of the system 100 and may process measurement data. The processing apparatus 140 may be connected over a local connection 141 to the network 150 and over a local connection 122 to a router 120. The processing apparatus 140 is configured to process the data, control quality of service, provide synchronization, collect data on user behavior, and enable service system setup and buffering, for example.

In an embodiment, the system 100 comprises an external apparatus 160 configured to be connectable to a cloud server apparatus 130 over a local connection 161 and network 150. The external apparatus 160 may access the portable apparatus generated data of plurality of different users via a cloud service provided by the cloud service server 130.

In an embodiment, the system 100 comprises a server apparatus 130, which comprises a storage device 131 for storing and providing service data, service metrics and subscriber information, over data connection 151. The service data may comprise configuration data, account creation data and measurement data, for example. The service metrics may comprise service operator information for use in both user identification and preventing service abuse.

In an embodiment, a proprietary application in the portable apparatus 110 or the processing apparatus 140 may be a client application of a service whose server application is running on the server apparatus 130 of the system 100. The proprietary application may capture the user input data for the service and provide the user output data, for the service. In an embodiment, configuration information between the processing apparatus 140, the portable apparatus 110 and the system server 130 is transceived via the first connections 112, 121, 122, 141, 150, 151, 152 automatically. Thus the user of the portable apparatus 110 may not need to do any control for the service. The system server 130 may also maintain account creation process details for the service, such as attaching new apparatuses to the system 100.

In an embodiment, the portable apparatus 110 may generate an estimate of a pulse based on received noisy biometric information. In addition or alternatively to the pulse estimation, the portable apparatus 110 may also generate oxygen saturation (SpO2) value, respiratory rate (RR) and blood sugar concentration, for example. Different applications may require different wavelengths and usage of several wavelengths and even several photo sensors. Embodiments of the invention may still be applied.

In an embodiment, a measured variable may vary but as long as the biometric information enables determination of a frequency and an amplitude model, the algorithm may be used to generate an estimate of the variable.

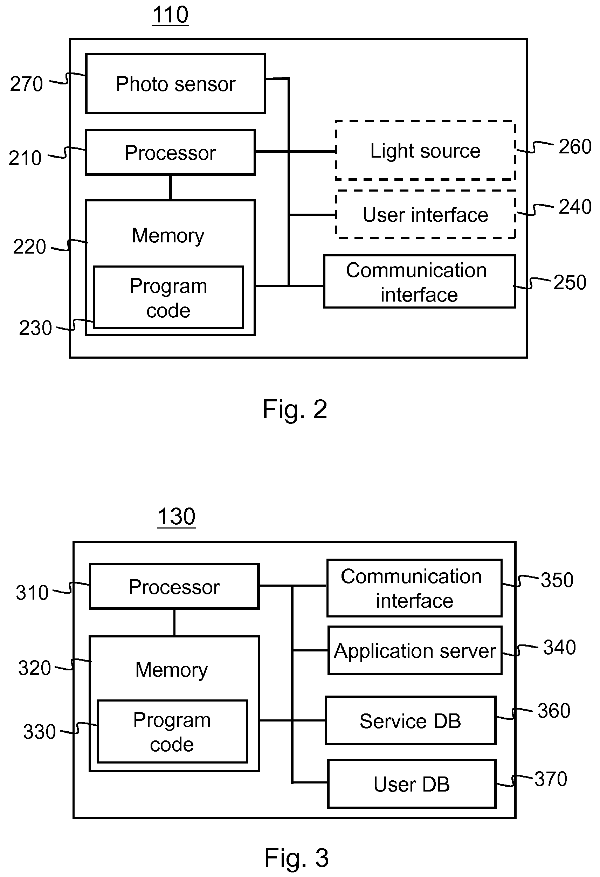

Fig. 2 presents an example block diagram of a portable apparatus 110 in which various embodiments of the invention may be applied. The portable apparatus 110 may be a portable device, a user wearable device, or a tag, or other communication device comprising a communication interface, light source and a photo sensor. All elements described in Fig. 2 are not necessary to be implemented in the same apparatus 110. For example a light source 260 and a user interface 240 are not necessary in the apparatus 110. The light source 260 may be implemented in a separate device, for example. The user interface 240 may be implemented also in another device connected via a communication interface 250 to the apparatus 110. Such device may comprise a mobile phone, a smart phone, or a tablet, for example.

The general structure of the portable apparatus 110 comprises a user interface 240, a communication interface 250, a light source 260, a photo sensor 270, a processor 210, and a memory 220 coupled to the processor 210. The portable apparatus 110 further comprises software 230 stored in the memory 220 and operable to be loaded into and executed in the processor 210. The software 230 may comprise one or more software modules and can be in the form of a computer program product. Not all elements of Fig. 2 are necessary but optional for the portable apparatus 110, such as the user interface 240.

The photo sensor 270 may comprise a photodiode or a phototransistor, for example.

The processor 210 may be, e.g., a central processing unit (CPU), a microprocessor, a digital signal processor (DSP), a graphics processing unit, or the like. Fig. 2 shows one processor 210, but the portable apparatus 110 may comprise a plurality of processors.

The memory 220 may be for example a non-volatile or a volatile memory, such as a read-only memory (ROM), a programmable read-only memory (PROM), erasable programmable read-only memory (EPROM), a random-access memory (RAM), a flash memory, a data disk, an optical storage, a magnetic storage, a smart card, or the like. The portable apparatus 110 may comprise a plurality of memories. The memory 220 may be constructed as a part of the portable apparatus 110 or it may be inserted into a slot, port, or the like of the portable apparatus 110 by a user. The memory 220 may serve the sole purpose of storing data, or it may be constructed as a part of an apparatus serving other purposes, such as processing data.

The user interface 240 that is optional for the apparatus 110 may comprise circuitry for receiving input from a user of the portable apparatus 110, e.g., via a keyboard, a touchpad, a motion sensor, a touch-screen of the portable apparatus 110, speech recognition circuitry, gesture recognition circuitry or an accessory device, such as a headset or a remote controller, for example. Furthermore, the user interface 240 may comprise circuitry for providing output for the user via a display, a touch-sensitive display or a lightning device, for example.

The communication interface module 250 implements at least part of data transmission. The communication interface module 250 may comprise, e.g., a wireless or a wired interface module. The wireless interface may comprise such as a WLAN, Bluetooth, infrared (IR), radio frequency identification (RF ID), NFC, GSM/GPRS, CDMA, WCDMA, or LTE (Long Term Evolution) radio module. The wired interface may comprise such as universal serial bus (USB), HDMI, SCART or RCA, for example. The communication interface module 250 may be integrated into the portable apparatus 110, or into an adapter, card or the like that may be inserted into a suitable slot or port of the portable apparatus 110. The communication interface module 250 may support one radio interface technology or a plurality of technologies. The communication interface module 250 may support one wired interface technology or a plurality of technologies. The portable apparatus 110 may comprise a plurality of communication interface modules 250.

In an embodiment, the communication interface module 250 may comprise location modules for tracking location of the portable apparatus 110. Such location modules may comprise a module for satellite based global positioning system (GPS), a module for cellular based positioning system, a module for wireless non-cellular positioning system (e.g. Wi-Fi) or a module for hybrid positioning system, for example.

A skilled person appreciates that in addition to the elements shown in Fig. 2, the portable apparatus 110 may comprise other elements, such as microphones, speakers, sensors, cameras, as well as additional circuitry such as input/output (I/O) circuitry, memory chips, application-specific integrated circuits (ASIC), processing circuitry for specific purposes such as source coding/decoding circuitry, channel coding/decoding circuitry, ciphering/deciphering circuitry, and the like. Additionally, portable apparatus 110 may comprise a disposable or rechargeable battery (not shown) for powering when external power if external power supply is not available.

In an embodiment, the portable apparatus 110 comprises a sensor for providing metadata associated to the estimate of a pulse. The metadata may comprise at least one of the following: temperature information; pressure information; movement information; location information; and humidity information.

In an embodiment, the portable apparatus 110 comprises speech or gesture recognition means. Using these means, a pre-defined phrase or a gesture may be recognized from the speech or the gesture and translated into control information for the apparatus 110, for example.

The heart rate extraction algorithm configured to be running in the memory 220 controlled by the processor 210, utilizes first filtering such as data binning to reduce time domain input data into a set of bins with median representatives to decrease the effect of random noise of the measured and analog-to-digital converted (ADC) biometric signal provided by the photo sensor 270. Then, the algorithm calculates the derivatives of the medians in order to obtain a set of local maxima or a set of local minima, or both.

The heart rate is then obtained from a pair of local maxima with the highest weight, where the weight is composed of two parts: a normalized sum of peak and a fitness of a frequency model to the input data. Frequency models are generated from the occurrence frequencies determined by the inspected pairs of maxima. The weight calculated may vary between 0 and 1.

Finally, this model is matched to the input by combining the frequency model and the local maxima into a single graph, finding the lightest path starting from the maxima with the lowest index and maximizing, first, the number of edges between the frequency model and the input data and, then, minimizing the distances between the vertices of these edges.

Fig. 3 presents an example block diagram of a server apparatus 130 in which various embodiments of the invention may be applied.

The general structure of the server apparatus 130 comprises a processor 310, and a memory 320 coupled to the processor 310. The server apparatus 130 further comprises software 330 stored in the memory 320 and operable to be loaded into and executed in the processor 310. The software 330 may comprise one or more software modules and can be in the form of a computer program product.

The processor 310 may be, e.g., a central processing unit (CPU), a microprocessor, a digital signal processor (DSP), a graphics processing unit, or the like. Fig. 3 shows one processor 310, but the server apparatus 130 may comprise a plurality of processors.

The memory 320 may be for example a non-volatile or a volatile memory, such as a read-only memory (ROM), a programmable read-only memory (PROM), erasable programmable read-only memory (EPROM), a random-access memory (RAM), a flash memory, a data disk, an optical storage, a magnetic storage, a smart card, or the like. The server apparatus 130 may comprise a plurality of memories. The memory 320 may be constructed as a part of the server apparatus 130 or it may be inserted into a slot, port, or the like of the server apparatus 130 by a user. The memory 320 may serve the sole purpose of storing data, or it may be constructed as a part of an apparatus serving other purposes, such as processing data.

The communication interface module 350 implements at least part of data transmission. The communication interface module 350 may comprise, e.g., a wireless or a wired interface module. The wireless interface may comprise such as a WLAN, Bluetooth, infrared (IR), radio frequency identification (RF ID), GSM/GPRS, CDMA, WCDMA, or LTE (Long Term Evolution) radio module. The wired interface may comprise such as Ethernet or universal serial bus (USB), for example. The communication interface module 350 may be integrated into the server apparatus 130, or into an adapter, card or the like that may be inserted into a suitable slot or port of the server apparatus 130. The communication interface module 350 may support one radio interface technology or a plurality of technologies. Configuration information between the portable apparatus 110 and the system server 130 may be transceived using the communication interface 350.

Similarly, account creation information between the system server 130 and a service provider may be transceived using the communication interface 350.

An application server 340 provides application services e.g. relating to the user accounts stored in a user database 370 and to the service information stored in a service database 360. The service information may comprise content information, content management information or metrics information, for example.

A skilled person appreciates that in addition to the elements shown in Fig. 3, the server apparatus 130 may comprise other elements, such as microphones, displays, as well as additional circuitry such as input/output (I/O) circuitry, memory chips, application-specific integrated circuits (ASIC), processing circuitry for specific purposes such as source coding/decoding circuitry, channel coding/decoding circuitry, ciphering/deciphering circuitry, and the like.

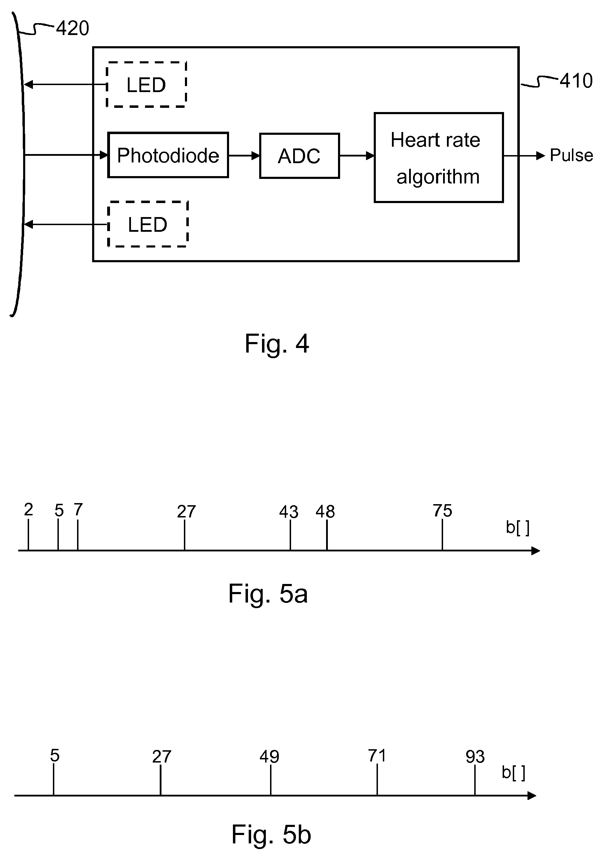

Fig. 4 presents an example block diagram of a portable apparatus 410 for generating an estimate of a pulse in which various embodiments of the invention may be applied.

An input signal may be obtained via photoplethysmography (PPG). A time domain heart rate recognition algorithm for a noisy signal may be implemented for the input signal, wherein the approximate heart rate is obtained by finding the most likely heart beat difference.

The heart beats are measured with a non-invasive portable apparatus 410, such as measurement device that senses arterial blood volume changes from a human via a photodiode and two light emitting diodes (LEDs). Unlike the most common approaches that utilize electrocardiography (ECG), the photodiode obtains the heart beats via the arterial blood volume changes that are caused by the pumping actions of the heart. Furthermore, since the pressure changes are not immediate pulses like the bioelectric activity in ECG, the signal of interest is more like a small hill than a sharp peak. Regardless, the time difference between these small hills and thus the heart rate can be approximated by comparing the peaks of the hills, or the local maxima, of the input signal.

Using light emitting diodes (LEDs) and a photodiode is a particularly convenient noninvasive measurement method. Typically it utilizes a pair of small light-emitting diodes (LEDs) transmitting light facing the patient's body 420, and measuring the reflected light beams using the photodiode. The received signal of the photodiode is analog-to-digital converted (ADC) using the ADC before processed by the heart rate algorithm. The outcome of the algorithm is the generated estimate of the pulse.

The light-emitting diodes may be implemented in a separate device than the photodiode, for example.

The light-emitting diodes (LED's) may be of different colors and wavelengths, depending on the measured value.

The monitored signal fluctuates in time with the heart beat because the arterial blood vessels expand and contract with each heartbeat.

Fig. 5a presents a schematic picture of binned input data peaks with median representatives in which various embodiments of the invention may be applied.

The heart rate algorithm takes a noisy input signal x[ ] with size N and produces a single heart rate estimate f(hr). First, the random input noise is diminished via data binning with median representatives, where every Nbin samples are replaced with their median. Thus, we obtain the binned input data b[ ] with size N/Nbin, as presented in Fig. 5a. The binned input data b[ ] is shown with seven bins represented by their medians: 2, 5, 7, 27, 43, 48, and 75, respectively.

Second, the local maxima (peaks) of the reduced input b[ ] are determined by taking the derivatives of the binned data and finding the peaks by inspecting the changes in the derivatives. The sizes of the peaks are chosen as the difference between the local maximum and the preceding local minimum in order to find the total height of the blood volume change. Clearly, the larger the peak, the less likely it is normal and uninteresting data variation.

Third, the obtained peaks are weighted in order to find the peaks that can be expected to belong to a valid heart rate f(hr). The weights W(ps, pt) are calculated for every peak pair (ps, pt), and it is defined as:

$$W(ps, pt) = aWp(ps, pt) + bWm(ps, pt)$$

where Wp(ps, pt) is the ratio of the sum of the peak sizes to the sum of the two largest peak sizes (in range [0, 1]), Wm(ps, pt) is the fitness of the heart rate generated by the two peaks ps and pt to the input data, and a and b are factors that determine the significance of each of the weights. Values a and b are selected so that a+b=1 and the selection of a and b may be done by the algorithm. The algorithm may use history data of earlier calculations to adjust a and b, for example.

In an embodiment, weight values Wp(ps, pt) are calculated for every possible peak pair and each calculated weight value Wp(ps, pt) may vary between 0 and 1 and are comparable with each other.

Fig. 5b presents a schematic picture of model input data peaks in which various embodiments of the invention may be applied.

The fitness of the heart rate generated by the inspected peaks ps and pt is calculated as follows. First, we generate model peaks as in Fig. 5b such that there are two model peaks at the locations of ps and pt, and the rest are placed at the constant interval determined by the location difference of the two inspected peaks, which corresponds to the tentative heart rate.

In Fig. 5b the model input data peaks are calculated with locations of ps and pt, respectively at 5 and 27. The difference of the used ps and pt is 22 and thus the model peaks are generated evenly as 5, 27, 49, 71 and 93. They are illustrated in Fig. 5b.

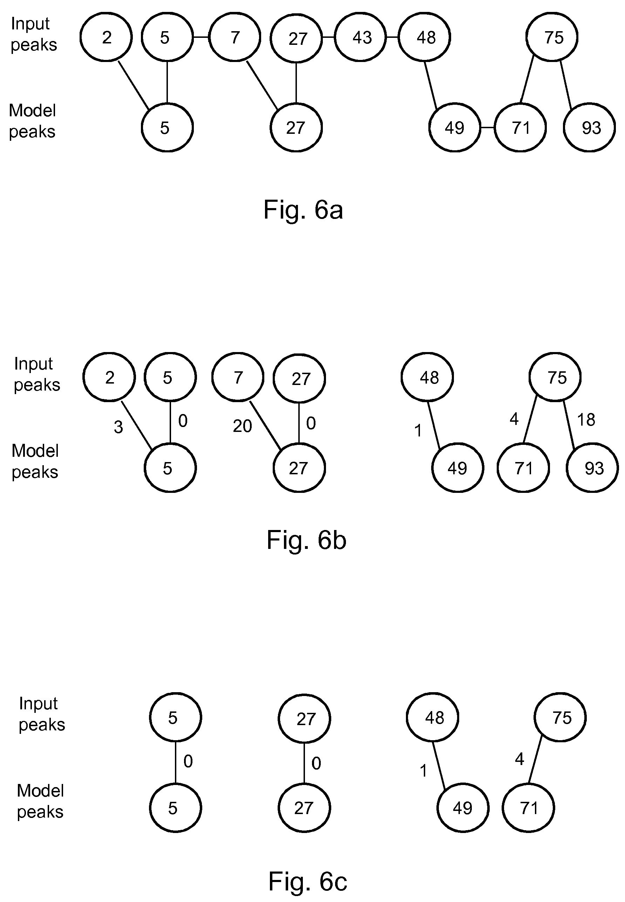

Fig. 6a presents a schematic picture of a graph representation with a lightest path using input data peaks (upper row) and model data peaks (lower row) in which various embodiments of the invention may be applied.

The input peaks and the model peaks are combined into a single graph, where the peaks originating from the input data and the generated model are connected to the peak which is closest to it and, if possible, originally from the other set, as shown in Fig. 6a. This maximizes the number of transitions between the two sets of peaks and, thus, the number of paired input and model peaks.

Fig. 6b presents a schematic picture of a graph representation including only interesting set switches in which various embodiments of the invention may be applied. Connections of peaks within the same set are removed and the distances between the peaks (local maxima) are also illustrated by numbers, extending from 0 to 20.

Fig. 6c presents a schematic picture of a graph representation with chosen minimal distance peak pairs in which various embodiments of the invention may be applied.

Third, the transitions between the two sets (input peaks/model peaks) which are both closest to each other and do not repeat any peaks (local maxima), are selected. Hence, the total weight Wm(ps, pt) contains the maximum number of unique matches between the two sets of peaks (input peaks/model peaks) such that their total difference is the smallest possible.

In the example illustration of Fig. 6c, the total distance on peak pair (5, 27) is thus:

$$d(5,5)+d(27,27)+d(48,49)+f(71,75)=0+0+1+4=5$$

The sum of these differences is modified into a weight with a value in range [0, 1] by utilizing the fact that the largest distance between two peaks of the graph representation is the constant difference between two consecutive model peaks.

Since both Wp(ps, pt) and Wm(ps, pt) are in range [0, 1], a comparable total weight W(ps, pt) may be obtained by choosing the factors a and b such that a + b = 1. Finally, the best heart rate estimate f(hr) is generated from the difference of the input peaks ps and pt with the largest weight.

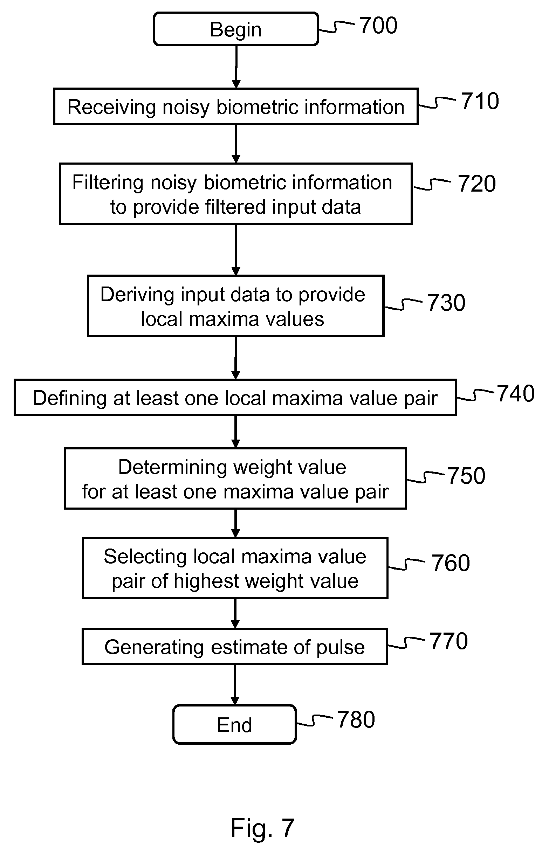

Fig. 7 shows a flow diagram showing operations of a portable apparatus in accordance with an example embodiment of the invention. In step 700, the method is started. In step 710, a noisy biometric information is received using a photo sensor. In step 720, the noisy biometric information is filtered for reducing noise. In step 730, the binned input data is derived to provide local maxima values of the binned input data. In step 740, a plurality of local maxima value pairs are defined. In step 750, a weight value is determined for the local maxima value pairs. In step 760, a local maxima value pair of the highest weight value is selected. In step 770, an estimate of a pulse is generated based on the difference of the local maxima values of the selected local maxima value pair. The method is ended in step 780.

No matter noisy biometric information is disclosed as captured from human body, also animals or any other living creature could be the source.

No matter noisy biometric information is disclosed as a starting point, also other sources of information may be used. Such information may comprise, for example, ventilation system measurement information, heating system information, water pipe related information, movement related information, audio information, and visual information. Any information that may be modeled by a frequency and an amplitude model may be used.

Various embodiments have been presented. It should be appreciated that in this document, words comprise, include and contain are each used as open-ended expressions with no intended exclusivity. If desired, the different functions discussed herein may be performed in a different order and/or concurrently with each other. Furthermore, if desired, one or more of the above-described functions may be optional or may be combined. Although various aspects of the invention are set out in the independent claims, other aspects of the invention comprise other combinations of features from the described embodiments and/or the dependent claims with the features of the independent claims, and not solely the combinations explicitly set out in the claims.

The foregoing description has provided by way of non-limiting examples of particular implementations and embodiments of the invention a full and informative description of the best mode presently contemplated by the inventors for carrying out the invention. It is however clear to a person skilled in the art that the invention is not restricted to details of the embodiments presented above, but that it can be implemented in other embodiments using equivalent means or in different combinations of embodiments without deviating from the characteristics of the invention.

Furthermore, some of the features of the above-disclosed embodiments of this invention may be used to advantage without the corresponding use of other features. As such, the foregoing description shall be considered as merely illustrative of the principles of the present invention, and not in limitation thereof. Hence, the scope of the invention is only restricted by the appended patent claims.

**Claims**

1. A portable apparatus (110) comprising:

> a communication interface (250) for communicating with a wireless network;
> a photo sensor (270) for receiving non-invasive biometric information of a user;
> a light source (260);
> at least one processor (210); and
> at least one memory (220) including computer program code (230); the at least one memory and the computer program code configured to, with the at least one processor, cause the portable apparatus to:

>> transmit a light beam using the light source;
>> receive noisy biometric information using the photo sensor, wherein the received noisy biometric information comprises a reflection of the transmitted light beam;
filter the noisy biometric information to provide filtered input data for reducing noise;
derive the input data to provide local maxima values of the input data;
**characterized in that** the portable apparatus is further configured to:

define at least one local maxima value pair;
determine a weight value for the at least one local maxima value pair;
select a local maxima value pair of the highest weight value; and
generate an estimate of a pulse based on the selected local maxima value pair.

2. The portable apparatus of claim 1, wherein filtering the noisy biometric information comprises at least one of the following:

resample the biometric information;
bin the biometric information;
noise reduce filter the biometric information;
all-pass filter (APF) the biometric information;
low-pass filter (LPF) the biometric information;
high-pass filter (HPF) the biometric information; and
band-pass filter (BPF) the biometric information.

3. The portable apparatus of claim 1 or 2, wherein the at least one memory and the computer program code configured to, with the at least one processor, cause the portable apparatus to:

generate an estimate of a pulse based on the difference of the local maxima values of the selected local maxima value pair.

4. The portable apparatus of any of claims 1 to 3, wherein the portable apparatus being a wearable apparatus comprising a light source device and a photo sensor device.

5. The portable apparatus of any of claims 1 to 4, wherein the photo sensor comprises a photodiode.

6. The portable apparatus of any of claims 1 to 5, wherein the noisy biometric information is received from a human body (420).

7. The portable apparatus of any of claims 1 to 6, wherein the light source comprises a light emitting diode.

8. The portable apparatus of any of claims 1 to 7, wherein the at least one memory and the computer program code configured to, with the at least one processor, cause the portable apparatus to:

transmit the estimate of the pulse to a server apparatus over a wireless network.

9. The portable apparatus of any of claims 1 to 8, wherein the at least one memory and the computer program code configured to, with the at least one processor, cause the portable apparatus to:

provide metadata associated to the estimate of a pulse.

10. The portable apparatus of claim 9, wherein the metadata comprising at least one of the following:

temperature information;
pressure information;
movement information;
location information; and
humidity information.

11. The portable apparatus of any of claims 1 to 10, wherein the received noisy biometric information comprises information on arterial blood volume changes caused by pumping actions of a heart.

**12.** The portable apparatus of any of claims 1 to 11, wherein the at least one memory and the computer program code configured to, with the at least one processor, cause the portable apparatus to:

filter the noisy biometric information to provide filtered input data for reducing noise;
derive the filtered input data to provide local maxima values; and
define a size of the local maxima value as a difference between the local maxima value and a preceding local minimum value of the filtered input data.

**13.** A system (100) comprising:

a portable apparatus (110) of any of claims 1 to 12; and
a server apparatus (130) comprising:

a communication interface (350) for communicating with a wireless network;
at least one processor (310); and
at least one memory (320) including computer program code (330);

the at least one memory and the computer program code configured to,
with the at least one processor, cause the server apparatus to:

receive an estimate of a pulse;
maintain user profile information for users of a server system service, comprising;
trigger an action based on the received estimate of the pulse and the user profile information; and
transmit the estimate of the pulse to a remote apparatus over the wireless network in response to the triggered action.

**14.** A method comprising:

transmit a light beam using a light source;
receiving noisy biometric information using a photo sensor, wherein the received noisy biometric information comprises a reflection of the transmitted light beam;
filtering the noisy biometric information to provide filtered input data for reducing noise;
deriving the input data to provide local maxima values of the input data;
**characterized in**
defining at least one local maxima value pair;
determining a weight value for the at least one local maxima value pair;
selecting a local maxima value pair of the highest weight value; and
generating an estimate of a pulse based on the selected local maxima value pair.

**15.** A computer program embodied on a computer readable medium comprising computer executable program code, which when executed by at least one processor of a portable apparatus, comprising a communication interface for communicating with a wireless network, a photo sensor and a light source, causes the portable apparatus to:

transmit a light beam using a light source;
receive noisy biometric information using a photo sensor, wherein the received noisy biometric information comprising a reflection of the transmitted light beam;
filter the noisy biometric information to provide filtered input data for noise reduction;
derive the input data to provide local maxima values of the input data;
**characterized in that** the portable apparatus is further configured to:

define at least one local maxima value pair;
determine a weight value for the at least one local maxima value pair;
select a local maxima value pair of the highest weight value; and
generate an estimate of a pulse based on the selected local maxima value pair.

**EP 2 848 194 B1**

**Patentansprüche**

1. Tragbare Vorrichtung (110) aufweisend:

   eine Kommunikationsschnittstelle (250) zum Kommunizieren mit einem drahtlosen Netzwerk;
   einen Fotosensor (270) zum Empfangen von nicht invasiver biometrischer Information eines Benutzers;
   eine Lichtquelle (260);
   wenigstens einen Prozessor (210); und
   wenigstens einen Speicher (220), der einen Computerprogrammcode (230) aufweist;
   wobei der wenigstens eine Speicher und der Computerprogrammcode eingerichtet sind, zusammen mit dem wenigstens einen Prozessor, die tragbare Vorrichtung zu veranlassen:

   einen Lichtstrahl zu senden unter Verwendung der Lichtquelle;
   rauschende biometrische Information unter Verwendung des Fotosensors zu empfangen, wobei die empfangene rauschende biometrische Information eine Reflektion des gesendeten Lichtstrahls aufweist;
   die rauschende biometrische Information zu filtern, um gefilterte Eingangsdaten zum Vermindern des Rauschens bereitzustellen;
   die Eingangsdaten abzuleiten, um lokale Maximalwerte der Eingangsdaten bereitzustellen;
   **dadurch gekennzeichnet, dass** die tragfähige Vorrichtung ferner eingerichtet ist:

   wenigstens ein lokales Maximalwertpaar zu definieren;
   einen Gewichtswert für das wenigstens eine lokale Maximalwertpaar zu bestimmen;
   ein lokales Maximalwertpaar des höchsten Gewichtswertes auszuwählen; und einen Schätzwert eines Impulses zu erzeugen, der auf dem ausgewählten lokalen Maximalwertpaar beruht.

2. Tragbare Vorrichtung nach Anspruch 1, wobei das Filtern der rauschenden biometrischen Information wenigstens eines der folgenden aufweist:

   Erneut abtasten der biometrischen Information;
   Auslagern der biometrischen Information;
   Filtern der biometrischen Information unter Verminderung des Rauschens;
   Pass-filtern (APF) der biometrischen Information;
   Tiefpass-filtern (LPF) der biometrischen Information;
   Hochpass-filtern (HPF) der biometrischen Information; und
   Bandpass-filtern (BPF) der biometrischen Information.

3. Tragbare Vorrichtung nach Anspruch 1 oder 2, wobei der wenigstens eine Speicher und der Computerprogrammcode eingerichtet sind, zusammen mit dem wenigstens einen Prozessor die tragbare Vorrichtung zu veranlassen:

   einen Schätzwert eines Impulses zu erzeugen, der auf der Differenz der lokalen Maximalwerte des ausgewählten lokalen Maximalwertpaares beruht.

4. Tragbare Vorrichtung nach einem der Ansprüche 1 bis 3, wobei die tragbare Vorrichtung eine tragbare Vorrichtung ist, die eine Lichtquellenvorrichtung und eine Fotosensorvorrichtung aufweist.

5. Tragbare Vorrichtung nach einem der Ansprüche 1 bis 4, wobei der Fotosensor eine Fotodiode aufweist.

6. Tragbare Vorrichtung nach einem der Ansprüche 1 bis 5, wobei die rauschende biometrische Information von einem menschlichen Körper (420) erhalten wird.

7. Tragbare Vorrichtung nach einem der Ansprüche 1 bis 6, wobei die Lichtquelle eine lichtemittierende Diode umfasst.

8. Tragbare Vorrichtung nach einem der Ansprüche 1 bis 7, wobei der wenigstens eine Speicher und der Computerprogrammcode eingerichtet sind, zusammen mit dem wenigstens einen Prozessor die tragbare Vorrichtung zu veranlassen:

   den Schätzwert des Impulses an eine Servervorrichtung über ein drahtloses Netzwerk zu senden.

13

9. Tragbare Vorrichtung nach einem der Ansprüche 1 bis 8, wobei der wenigstens eine Speicher und der Computerprogrammcode eingerichtet sind, zusammen mit dem wenigstens einen Prozessor die tragbare Vorrichtung zu veranlassen:

Metadaten bereitzustellen, die mit dem Schätzwert eines Impulses verknüpft sind.

10. Tragbare Vorrichtung nach Anspruch 9, wobei die Metadaten wenigstens eines der folgenden aufweisen:

Temperaturinformation;
Druckinformation;
Bewegungsinformation;
Ortsinformation; und
Feuchtigkeitsinformation.

11. Tragbare Vorrichtung nach einem der Ansprüche 1 bis 10, wobei die empfangene rauschende biometrische Information eine Information über die Volumenänderung des arteriellen Blutes enthält, die durch Pumpaktionen eines Herzens verursacht werden.

12. Tragbare Vorrichtung nach einem der Ansprüche 1 bis 11, wobei der wenigstens eine Speicher und der Computerprogrammcode konfiguriert sind, zusammen mit dem wenigstens einen Prozessor die tragbare Vorrichtung zu veranlassen:

die rauschende biometrische Information zu filtern, um gefilterte Eingangsdaten zum Vermindern des Rauschens bereitzustellen;
die gefilterten Eingangsdaten abzuleiten, um lokale Maximalwerte bereitzustellen; und
eine Größe des lokalen Maximalwertes als eine Differenz zwischen dem lokalen Maximalwert und einem vorangehenden lokalen Minimalwert der gefilterten Eingangsdaten zu definieren.

13. System (100), aufweisend:

eine tragbare Vorrichtung (110) gemäß einem der Ansprüche 1 bis 12; und
eine Servervorrichtung (130), aufweisend:

eine Kommunikationsschnittstelle (350) zum Kommunizieren mit einem drahtlosen Netzwerk;
wenigstens einen Prozessor (310); und
wenigstens einen Speicher (320), der einen Computerprogrammcode (330) aufweist;

wobei der wenigstens eine Speicher und der Computerprogrammcode konfiguriert sind, zusammen mit dem wenigstens einen Prozessor die Servervorrichtung zu veranlassen:

einen Schätzwert eines Impulses zu erhalten;

eine Benutzerprofilinformation für Benutzer eines Serversystemdienstes aufrechtzuerhalten, aufweisend:

Triggern einer, auf dem erhaltenen Schätzwert des Impulses und der Benutzerprofilinformation beruhenden Aktion; und
Senden des Schätzwertes des Impulses an eine entfernte Vorrichtung über das drahtlose Netzwerk in Reaktion auf die getriggerte Aktion.

14. Verfahren, aufweisend:

Senden eines Lichtstrahls unter Verwendung einer Lichtquelle;
Empfangen von rauschender biometrischer Information unter Verwendung eines Fotosensors, wobei die empfangene rauschende biometrische Information eine Reflektion des gesendeten Lichtstrahls aufweist;
Filtern der rauschenden biometrischen Information, um gefilterte Eingangsdaten zum Vermindern des Rauschens bereitzustellen;
Ableiten der Eingangsdaten, um lokale Maximalwerte der Eingangsdaten bereitzustellen;
**gekennzeichnet durch**

Definieren wenigstens eines lokalen Maximalwertpaares;

Bestimmen eines Gewichtswertes für das wenigstens eine lokale Maximalwertpaar;

Auswählen eines lokalen Maximalwertpaares des höchsten Gewichtswerts; und

Erzeugen eines Schätzwertes eines Impulses, der auf dem ausgewählten lokalen Maximalwertpaar beruht.

15. Computerprogramm, enthalten auf einem computerlesbaren Medium, aufweisend vom Computer ausführbaren Programmcode, der wenn er ausgeführt wird, durch wenigstens einen Prozessor einer tragfähigen Vorrichtung aufweisend eine Kommunikationsschnittstelle zum Kommunizieren mit einem drahtlosen Netzwerk, einem Fotosensor und einer Lichtquelle, die tragfähige Vorrichtung veranlasst:

einen Lichtstrahl unter Verwendung einer Lichtquelle zu senden;

rauschende biometrische Information unter Verwendung eines Fotosensors zu empfangen, wobei die empfangene rauschende biometrische Information eine Reflektion des gesendeten Lichtstrahls aufweist;

Filtern der rauschenden biometrischen Information, um gefilterte Eingangsdaten zur Rauschverminderung bereitzustellen;

Ableiten der Eingangsdaten, um lokale Maximalwerte der Eingangsdaten bereitzustellen;

**dadurch gekennzeichnet, dass** die tragfähige Vorrichtung ferner konfiguriert ist:

wenigstens ein lokales Maximalwertpaar zu definieren;

einen Gewichtswert für das wenigstens eine lokale Maximalwertpaar zu bestimmen;

ein lokales Maximalwertpaar des höchsten Gewichtswertes auszuwählen; und

einen Schätzwert eines Impulses zu erzeugen, der auf dem ausgewählten lokalen Maximalwertpaar beruht.

**Revendications**

1. Appareil portatif (110) comprenant :

une interface de communication (250) pour communiquer avec un réseau sans fil ;

un capteur optique (270) pour recevoir des informations biométriques non-invasives d'un utilisateur ;

une source de lumière (260) ;

au moins un processeur (210) ; et

au moins une mémoire (220) comprenant un code de programme informatique (230) ; ladite au moins une mémoire et le code de programme informatique étant configurés pour, avec ledit au moins un processeur, amener l'appareil portatif à :

émettre un faisceau lumineux en utilisant la source de lumière ;

recevoir des informations biométriques de bruit à l'aide du capteur optique, dans lequel les informations biométriques de bruit reçues comprennent une réflexion du faisceau lumineux émis ;

filtrer les informations biométriques de bruit pour fournir des données d'entrée filtrées pour réduire le bruit ;

dériver les données d'entrée pour fournir des valeurs de maxima locaux des données d'entrée ;

**caractérisé en ce que** l'appareil portatif est en outre configuré pour :

définir au moins une paire de valeurs de maxima locaux ;

déterminer une valeur de poids pour ladite au moins une paire de valeurs de maxima locaux ;

sélectionner une paire de valeurs de maxima locaux de la valeur de poids la plus élevée ; et

générer une estimation d'une pulsation sur la base de la paire de valeurs de maxima locaux sélectionnée.

2. Appareil portatif selon la revendication 1, dans lequel le filtrage des informations biométriques de bruit comprend au moins une des opérations suivantes consistant à :

ré-échantillonner les informations biométriques ;

rejeter les informations biométriques ;

traiter avec un filtre de réduction du bruit les informations biométriques ;

traiter avec un filtre passe-tout (APF) les informations biométriques ;

traiter avec un filtre passe-bas (LPF), les informations biométriques ;

traiter avec un filtre passe-haut (HPF) les informations biométriques ; et

traiter avec un filtre passe-bande (BPF) les informations biométriques.

**3.** Appareil portatif selon la revendication 1 ou 2, dans lequel ladite au moins une mémoire et le code de programme informatique sont configurés pour, avec ledit au moins un processeur, amener l'appareil portatif à :

générer une estimation d'une pulsation sur la base de la différence des valeurs de maxima locaux de la paire de valeurs de maxima locaux sélectionnée.

**4.** Appareil portatif de l'une quelconque des revendications 1 à 3, dans lequel l'appareil portatif est un appareil portatif comprenant un dispositif de source de lumière et un dispositif de capteur optique.

**5.** Appareil portatif selon l'une quelconque des revendications 1 à 4, dans lequel le capteur optique comprend une photodiode.

**6.** Appareil portatif selon l'une quelconque des revendications 1 à 5, dans lequel les informations biométriques de bruit sont reçues d'un corps humain (420).

**7.** Appareil portatif de l'une quelconque des revendications 1 à 6, dans lequel la source de lumière comprend une diode électroluminescente.

**8.** Appareil portatif de l'une quelconque des revendications 1 à 7, dans lequel ladite au moins une mémoire et le code de programme informatique sont configurés pour, avec ledit au moins un processeur, amener l'appareil portatif à :

transmettre l'estimation de la pulsation à un appareil de serveur sur un réseau sans fil.

**9.** Appareil portatif de l'une quelconque des revendications 1 à 8, dans lequel ladite au moins une mémoire et le code de programme informatique sont configurés pour, avec ledit au moins un processeur, amener l'appareil portatif à :

fournir des métadonnées associées à l'estimation d'une pulsation.

**10.** Appareil portatif selon la revendication 9, dans lequel les métadonnées comprenant au moins une des informations suivantes :

des informations de température ;
des informations de pression ;
des informations de mouvement ;
des informations de localisation ; et
des informations d'humidité.

**11.** Appareil portatif de l'une quelconque des revendications 1 à 10, dans lequel les informations biométriques de bruit reçues comprennent des informations sur les modifications du volume sanguin artériel provoquées par les actions de pompage d'un coeur.

**12.** Appareil portatif de l'une quelconque des revendications 1 à 11, dans lequel ladite au moins une mémoire et le code de programme informatique sont configurés pour, avec ledit au moins un processeur, amener l'appareil portatif à :

filtrer les informations biométriques de bruit pour fournir des données d'entrée filtrées pour réduire le bruit :

dériver les données d'entrée filtrées pour fournir des valeurs de maxima locaux, et
définir une taille de la valeur des maxima locaux en tant que différence entre la valeur des maxima locaux et une valeur minimale locale précédente des données d'entrée filtrés.

**13.** Système (100) comprenant :

un appareil portatif (110) selon l'une quelconque des revendications 1 à 12 ; et
un appareil de serveur (130), comprenant :

une interface de communication (350) pour communiquer avec un réseau sans fil ;

au moins un processeur (310) ; et
au moins une mémoire (320) comprenant un code de programme informatique (330) ;
ladite au moins une mémoire et le code de programme informatique étant configurés pour, avec ledit au moins un processeur, amener le dispositif de serveur à :

    recevoir une estimation d'une pulsation ;
    maintenir des informations de profil d'utilisateur pour des utilisateurs d'un service de système de serveur, comprenant les étapes consistant à ;

déclencher une action sur la base de l'estimation de la pulsation reçue et des informations de profil d'utilisateur ; et transmettre l'estimation de la pulsation à un appareil à distance sur le réseau sans fil en réponse à l'action déclenchée.

**14.** Procédé comprenant les étapes consistant à :

    émettre un faisceau lumineux en utilisant une source de lumière ;
    recevoir des informations biométriques de bruit en utilisant un capteur optique, dans lequel les informations biométriques de bruit reçues comprennent une réflexion du faisceau lumineux émis ;
    filtrer les informations biométriques de bruit pour fournir des données d'entrée filtrées pour réduire le bruit ;
    dériver les données d'entrée pour fournir des valeurs de maxima locaux des données d'entrée ; **caractérisé par** les étapes consistant à
    définir au moins une paire de valeurs de maxima locaux ;
    déterminer une valeur de poids pour ladite au moins une paire de valeurs de maxima locaux ;
    sélectionner une paire de valeurs de maxima locaux de la valeur de poids la plus élevée ; et
    générer une estimation d'une pulsation sur la base de la paire de valeurs de maxima locaux sélectionnée.

**15.** Programme informatique incorporé sur un support lisible par ordinateur comprenant un code de programme exécutable par ordinateur, lequel, lorsqu'il est exécuté par au moins un processeur d'un appareil portatif, comprenant une interface de communication pour communiquer avec un réseau sans fil, un capteur optique et une source lumineuse, amène l'appareil portatif à :

    émettre un faisceau lumineux en utilisant une source de lumière ;
    recevoir des informations biométriques de bruit en utilisant un capteur optique, dans lequel les informations biométriques de bruit reçues comprennent une réflexion du faisceau lumineux émis ;
    filtrer les informations biométriques de bruit pour fournir des données d'entrée filtrées pour une réduction du bruit ;
    dériver les données d'entrée pour fournir des valeurs de maxima locaux des données d'entrée ; **caractérisé en ce que** l'appareil portatif est en outre configuré pour :

        définir au moins une paire de valeurs de maxima locaux ;
        déterminer une valeur de poids pour ladite au moins une paire de valeurs de maxima locaux ;
        sélectionner paire de valeurs de maxima locaux de la valeur de poids la plus élevée ; et
        générer une estimation d'une pulsation sur la base de la paire de valeurs de maxima locaux sélectionnée.

Fig. 1

## 110

270 — Photo sensor

210 — Processor

220 — Memory

230 — Program code

260 — Light source

240 — User interface

250 — Communication interface

Fig. 2

## 130

310 — Processor

320 — Memory

330 — Program code

350 — Communication interface

340 — Application server

360 — Service DB

370 — User DB

Fig. 3

Fig. 4

Fig. 5a

Fig. 5b

Input
peaks

Model
peaks

Fig. 6a

Input
peaks

Model
peaks

Fig. 6b

Input
peaks

Model
peaks

Fig. 6c

Begin ⟿ 700

↓

Receiving noisy biometric information ⟿ 710

↓

Filtering noisy biometric information to provide filtered input data ⟿ 720

↓

Deriving input data to provide local maxima values ⟿ 730

↓

Defining at least one local maxima value pair ⟿ 740

↓

Determining weight value for at least one maxima value pair ⟿ 750

↓

Selecting local maxima value pair of highest weight value ⟿ 760

↓

Generating estimate of pulse ⟿ 770

↓

End ⟿ 780

Fig. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2009326395 A